**Europäisches Patentamt**

**European Patent Office**

**Office européen des brevets**

(19)

(11) Veröffentlichungsnummer: **0 201 737**
**A2**

(12) # EUROPÄISCHE PATENTANMELDUNG

(21) Anmeldenummer: **86105110.0**

(22) Anmeldetag: **14.04.86**

(51) Int. Cl.⁴: **A 61 B 17/58**

(30) Priorität: **07.05.85 CH 1932/85**
**03.10.85 CH 4280/85**

(43) Veröffentlichungstag der Anmeldung:
**20.11.86 Patentblatt 86/47**

(84) Benannte Vertragsstaaten:
**AT BE CH DE FR GB LI LU NL SE**

(71) Anmelder: **SYNTHES AG**
**Grabenstrasse 15**
**CH-7002 Chur(CH)**

(72) Erfinder: **Frigg, Robert**
**Fellerstrasse 9a**
**CH-3027 Bern(CH)**

(72) Erfinder: **Gysin, Paul**
**Brestenbergweg 7**
**CH-4437 Waldenburg(CH)**

(72) Erfinder: **Perren, Stephen M., Prof. Dr. med.**
**Dischmastrasse 22**
**CH-7260 Davos Dorf(CH)**

(72) Erfinder: **Jenny, Urs**
**Hauptstrasse 19**
**CH-4435 Niederdorf(CH)**

(74) Vertreter: **Lusuardi, Werther Giovanni, Dr.**
**Patentanwalt Stockerstrasse 8**
**CH-8002 Zürich(CH)**

(54) **Visierinstrument.**

(57) Das insbesondere für die distale Verriegelung der Marknagelung dienende Visierinstrument (10) besteht aus einem Handgriff (11) einer damit verbundenen Positionierhülse (12) und dem eigentlichen Visier (13), dessen Position zwischen einer Strahlenquelle (3) und einem Strahlenempfänger (2) durch einen Bildwandler (4) sichtbar auf einem Monitor (5) dargestellt werden kann.

Fig. 6

EP 0 201 737 A2

Croydon Printing Company Ltd.

1049/EU

## Visierinstrument

Die Erfindung bezieht sich auf ein Visierinstrument, insbesondere für chirurgische Zwecke, mit einem Handgriff und einer damit verbundenen Bohrhülse, dessen Verwendung, sowie auf ein Verfahren zur kontrollierten Durchdringung eines Materials in einer gewünschten Richtung mittels eines Werkzeugs.

Es sind bereits Vorrichtungen bekannt, bei denen eine Bohrlehre mittels eines Röntgenbildwandlers justiert und anschliessend in der gewünschten Position fixiert wird. Beispielsweise ist aus der CH-A5 635 998 ein distales Zielgerät für die Verriegelungsnagelung bekannt geworden, mit einem Zielkopf, der eine Bohrung zur Aufnahme einer Zielhülse aufweist. Die Zielkopfhalterung ist in einer Fassung angebracht, die mit dem Röntgengerät verbunden ist und von diesem abhängig ist. Wegen der dadurch bewirkten schwerfälligen Positionierung und Fixierung ist die Zielgenauigkeit unbefriedigend. Durch die fixe Anordnung ergibt sich zudem eine eingeschränkte Betriebsweise.

Es ist daher auch bekanntgeworden, ein distales Zielgerät zu schaffen, das unabhängig vom Röntgengerät verwendet werden kann, beispielsweise aus dem DE-U1 84 17 428, mit einem in einer Halterung liegenden, röntgenstrahlendurchlässigen Aufnahmekopf zur Aufnahme eines Bohrers oder eines Bohrdrahtes. Auch bei dieser verbesserten Vorrichtung verbleiben aber gewichtige Nachteile, insbesondere die Tatsache, dass der Zielvorgang in der Querbohrung des Verriegelungsnagels stattfindet, was eine starke Abdunkelung des Arbeitsfeldes und eine niedrige Bildauflösung bewirkt.

Schliesslich führt das Fehlen einer das Gewebe schützenden Bohrhülse zu einer Schädigung der Weichteile bei jedem Auswechseln des zu verwendenden Werkzeuges oder Instrumentes.

Alle bekannten Vorrichtungen sind zudem mit dem schwerwiegenden Mangel behaftet, während der Operation keine Ueberprüfung des Zielvorganges und entsprechende Korrekturen zu ermöglichen.

Hier will die Erfindung Abhilfe schaffen. Die Erfindung, wie sie in den Ansprüchen gekennzeichnet ist, löst die Aufgabe ein Visierinstrument der beschriebenen Art zu schaffen mit welchem auch während der Werkzeugbetätigung eine permanente Kontrolle und Korrektur der relativen Lage der Achse der Bohrhülse bezüglich einer gewünschten Richtung möglich ist und ein optimaler Gewebeschutz gewährleistet ist.

Die durch die Erfindung erreichten Vorteile sind im wesentlichen darin zu sehen, dass mittels eines einfachen, platzsparenden Visierinstruments höchste Präzision bei der Anwendung von Werkzeugen, insbesondere chirurgischer Art, erzielt werden kann und dass insgesamt der Zielvorgang wesentlich verkürzt werden kann, was beim Arbeiten in einem Röntgenstrahlfeld auch von der Arbeitssicherheit her bedeutsam ist. So konnte beispielsweise die Röntgenbestrahlung bei einer typischen Verriegelung der Marknagelung von mehreren Minuten auf etwa 10 Sekunden reduziert werden.

Durch Verlagerung des Visiervorgangs ausserhalb der Querbohrung des Verriegelungsnagels konnte zudem eine erheblich verbesserte Bildauflösung erreicht werden.

Im folgenden wird die Erfindung anhand einer lediglich einen Anwendungsfall darstellenden Zeichnung näher erläutert.

Es zeigt

Figur 1    in perspektivischer Darstellung das Operationsfeld bei einer Verriegelungsnagelung mit dem erfindungsgemässen Visierinstrument und der für den Visiervorgang benötigten Röntgenapparatur;

Figur 2a    eine Aufsicht des erfindungsgemässen Visierinstruments;

Figur 2b    eine Seitenansicht des erfindungsgemässen Visierintrumentes mit einem in die Bohrhülse eingeführten Stift zur Setzung des Positionspunktes;

Figur 3a    eine perspektivische Ansicht der erfindungsgemässen Instrumentenkonfiguration gemäss Figur 2b im positionierten aber noch nicht ausgerichteten Zustand;

Figur 3b    eine perspektivische Ansicht des erfindungsgemässen Visierinstrumentes im positionierten und im Strahlenfeld ausgerichteten Zustand mit dem in die Bohrhülse eingeführten Bohrer;

Figur 4a    eine schematische Darstellung des Strahlenfeldes einer Röntgenapparatur;

Figur 4b    einen Schnitt durch die Bohrhülse und das Visier des erfindungsgemässen Visierinstrumentes;

Figur 5a    eine Aufsicht des erfindungsgemässen Visierinstrumentes mit einer zusätzlichen Bohrbüchse für das Setzen der zweiten Verriegelungsschraube;

Figur 5b    eine Seitenansicht von links der Figur 5a;

Figur 5c    eine Seitenansicht von oben der Figur 5a;

Figur 6     eine perspektivische Ansicht des Visierinstrumentes gemäss Fig. 5 mit eingesetzter erster Verriegelungsschraube;

Figur 7a    ein Monitorbild des nicht ausgerichteten Visierinstrumentes gemäss Figur 6; und

Figur 7b    ein Monitorbild des ausgerichteten Visierinstrumentes gemäss Figur 6.

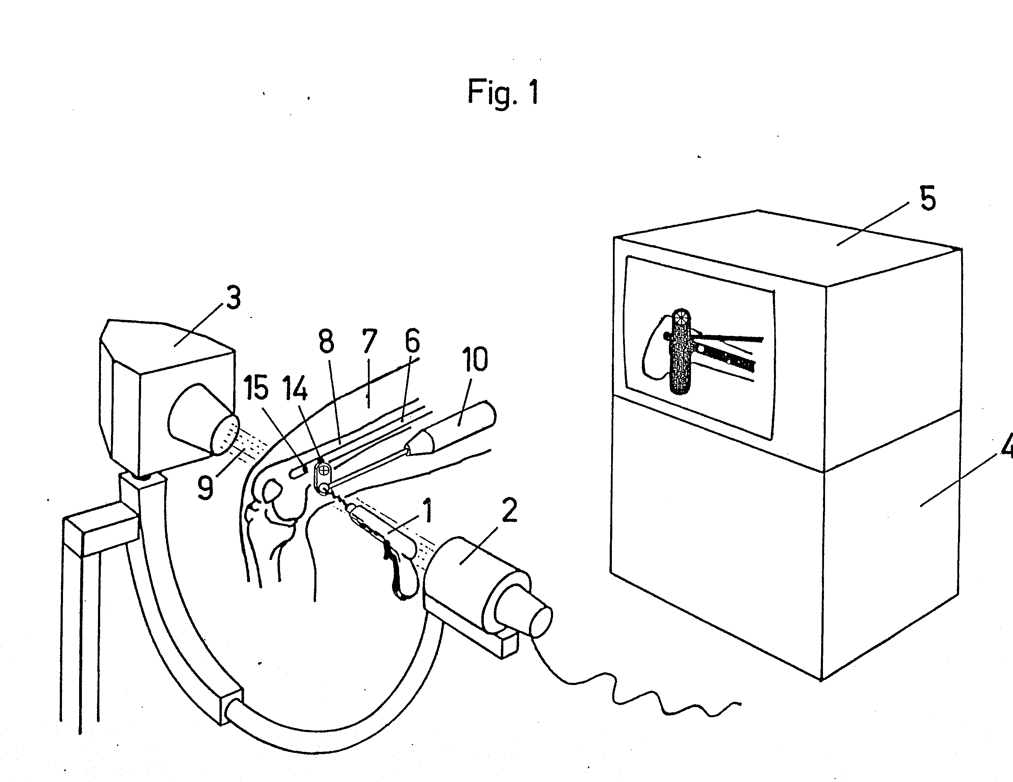

In Figur 1 ist schematisch die bei einer Verriegelungsnagelung verwendete Apparatur dargestellt, welche aus der Röntgenquelle 3, dem darauf abgestimmten Röntgenstrahlenempfänger 2 und einen an diesen angeschlossenen Bildwandler 4 mit
Monitor 5 besteht.

Im Strahlenfeld 9 der Röntgenapparatur befindet sich der
Oberschenkel 7 des Patienten mit dem bereits in die Markhöhle des Femur 8 eingeführten Verriegelungsnagel 6, sowie
der zur Durchbohrung der äusseren Corticalis angesetzte Bohrer 1 mit Visierinstrument 10.

Die relative Lage der Bohrerspitze zum Verriegelungsnagel 6
sowie die Ausrichtung des Visierinstruments zum Strahlenfeld
9 kann jederzeit auf dem Monitor 5 des Bildwandlers 4 verfolgt und nötigenfalls korrigiert werden.

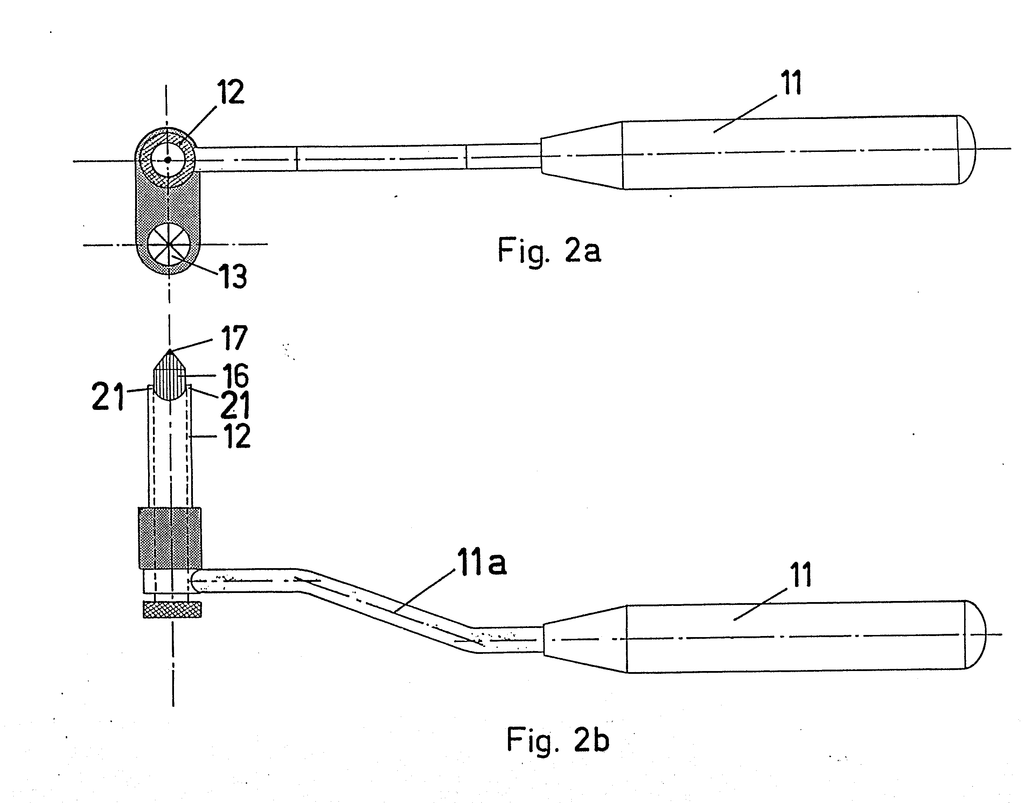

Das in Figur 2a und b dargestellte erfindungsgemässe Visierinstrument besteht aus einem Handgriff 11 an dem eine Bohrhülse 12 befestigt ist um die ein Fadenkreuzvisier 13 verschwenkt werden kann.

Die Bohrhülse 12 besteht im wesentlichen aus einem zwei Zähne 21 aufweisenden Hohlzylinder, der einen Stift 16 aus Kunststoff aufnehmen kann, an dessen Spitze eine Metallkugel 17 eingebettet ist. Das eigentliche Visier 13 besteht aus zwei in verschiedenen Ebenen liegenden gegeneinander verdrehten Fadenkreuzen oder aus zwei anderen geeigneten Visierbil-dern,beispielsweise einen Kreis und einen Punkt.

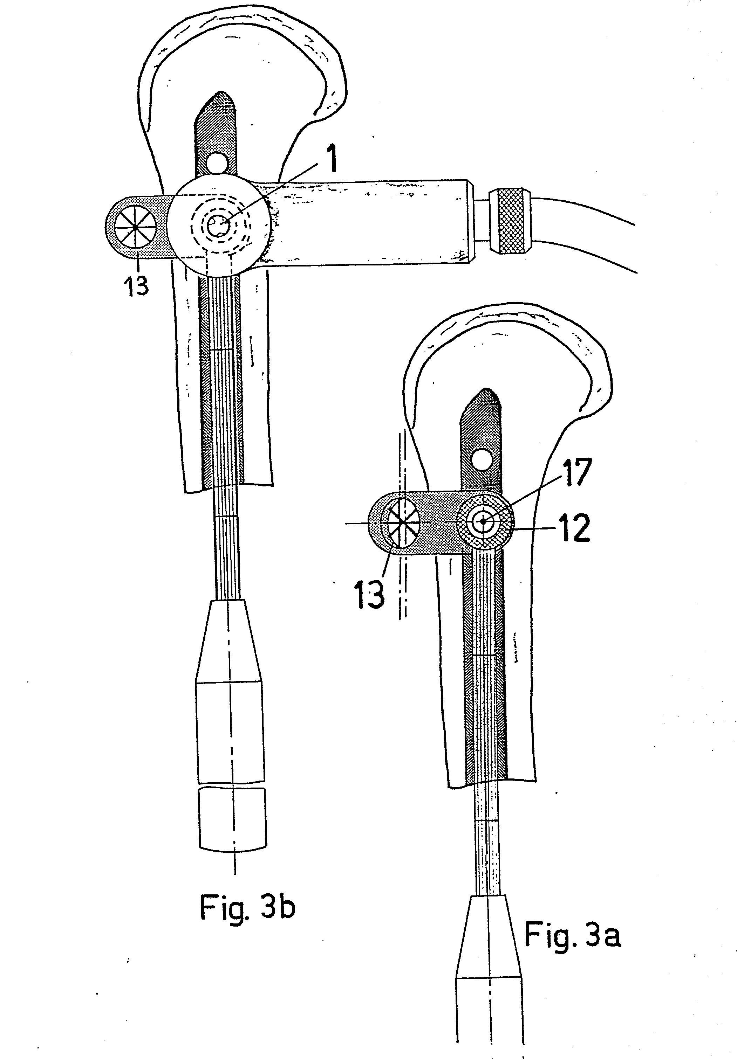

Nun soll anhand der Figuren 1, 3a und 3b die Operationstechnik bei der Verriegelung der Marknagelung mit dem erfindungsgemässen Visierinstrument 10 beschrieben werden.

Bevor das erfindungsgemässe Visierinstrument 10 bei der distalen Verriegelung des Marknagels 6 zur Anwendung kommt wird die relative Lage des Marknagels 6 zur Röntgenapparatur 2,3 derart ausgerichtet, dass die Verriegelungslöcher 14,15 auf dem Monitor 5 rund abgebildet werden. Dies ist dann der Fall, wenn die Achsen der Verriegelungslöcher 14,15 parallel zum Strahlenfeld 9 liegen. Das zu verriegelnde Loch sollte dabei in der Mitte des unteren Bildrandes zu liegen kommen, damit das Fadenkreuz des Visiers 13 in die Bildmitte zu stehen kommt. Hierauf wird der Oberschenkel 7 mit einer Strahlenschutzmatte bedeckt, welche eine dem Operationsfeld entsprechende Oeffnung besitzt.

Nachdem unter Bildverstärker-Kontrolle ein genau über dem Verriegelungsloch liegender Hautschnitt bis auf die Knochenoberfläche geführt worden ist kann das Visierinstrument 10, mit eingeschobenem Stift 16, durch den Hautschnitt eingeführt werden (Fig.3a).

Der Stift 16 vereinfacht das Einführen des Visierinstrumentes durch die Weichteile, dank seiner kugelförmigen Spitze.

Nach erfolgter Einführung wird die Spitze des Stiftes 16 mit dem kugelförmigen Referenzkörper 17 über das Zentrum des Verriegelungsloches gesetzt. Dieser Arbeitsschritt ist in Figur 3a dargestellt und zeigt den exakt im Zentrum der Bohrhülse 12 liegenden Referenzkörper 17.

0201737

Das Visier 13 soll in diesem Zeitpunkt noch nicht beachtet werden. Wenn der Referenzkörper 17 genau im Zentrum des Verriegelungsloches liegt wird das Visierinstrument fest auf den Femur 8 gedrückt. Die Zähne 21 der Bohrhülse 12 verhindern ein Abrutschen des Visierinstrumentes 10 vom Femur 8. In diesem Stadium der Operation ist darauf zu achten, dass das Visierinstrument 10 nicht um die Bohrhülse 12 gedreht wird, da die als Gewebeschutz dienende Bohrhülse 12 mit den beiden Zähnen und nicht mit einer zentrischen Spitze auf dem Femur 8 aufliegt.

Nach erfogter Positionierung des Visierinstrumentes 10 wird der Stift 16 entfernt und eine 4,5mm Bohrbüchse in die Bohrhülse 12 eingesetzt. In diese 4,5mm Bohrbüchse wird nun der 4,5mm Bohrer eingeführt, dessen Spitze exakt über dem Zentrum des Verriegelungsloches zu liegen kommt. Nun wird die Bohrrichtung unter dem Bildverstärker eingestellt. Dies geschieht, wie in Figur 3b dargestellt, indem das Visier 13 senkrecht zum Strahlenfeld 9 ausgerichtet wird. Jetzt kann die erste Kortikalis mit dem 4,5mm Bohrer aufgebohrt werden. Die Bohrrichtung kann laufend mit dem Bildverstärker überprüft werden und nötigenfalls durch Anpassung des Visierbildes korrigiert werden.

Wie in Figur 3b ersichtlich ist das Verriegelungsloch bei diesem Operationsschritt durch die Bohrmaschine verdeckt. Nach Durchbohrung der ersten Kortikalis wird der 4,5mm Bohrer aus der 4,5mm Bohrbüchse entfernt und eine 3,2mm Steckbohrbüchse bis auf die Gegenkortikalis eingeführt. Der Bildverstärker kann nun weggeschwenkt werden und die Gegenkortikalis mit dem 3,2mm Bohrer aufgebohrt werden. Nach Entfernung der 3,2mm Steckbohrbüchse wird die Schraubenlänge gemessen, ein Gewinde in die Gegenkortikalis geschnitten und die Verriegelungsschraube gesetzt.

Für das Setzen der zweiten Verriegelungsschraube wird die ganze beschriebene Prozedur grundsätzlich wiederholt.

Je nach Anwendungsfall sind ausser der im Anwendungsbeispiel erwähnten Röntgenapparatur auch andere Strahlenquellen 3 verwendbar, beispielsweise Ultraschall oder speziell für den Ausbildungsbetrieb auch gewöhnliches, sichtbares Licht.

Das mit der erfindungsgemässen Zielvorrichtung geführte Werkzeug 1 kann ausser dem im Beispiel erwähnten Bohrer auch aus einer Säge, Spritze oder einem anderen chirurgischen Instrument bestehen.

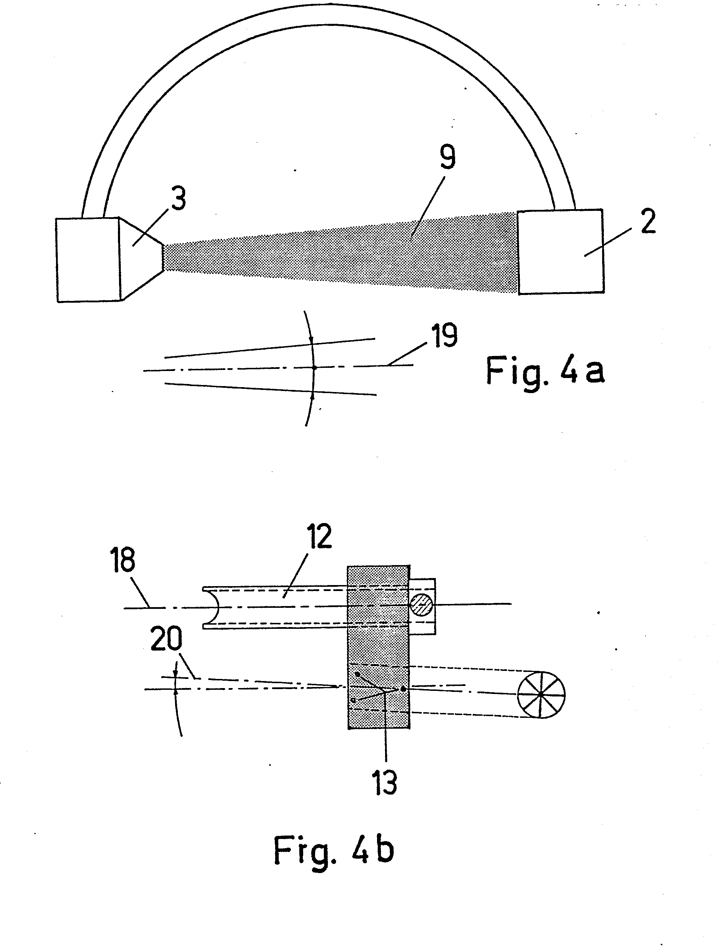

Obwohl sich die erfindungsgemässe Zielvorrichtung vorzugsweise für in der Orthopädie, Arthroskopie, Wirbelsäulenchirurgie und Punktion verwendete chirurgische Werkzeuge und Instrumente eignet, kann das dabei angewendete Verfahren auch für andere nichtchirurgische Zwecke verwendet werden. Um die Präzision des Visierinstrumentes 10 weiter zu erhöhen kann, wie in Figur 4b dargestellt, die in einer gemeinsamen Ebene liegende Achse 18 der Bohrhülse 12 mit der optischen Visierlinie 20 einen Winkel einschliessen um der Divergenz des Strahlenfeldes 9 (Fig.4a) Rechnung zu tragen. Diese präzionserhöhende Ausführungsform kann entweder fix oder justierbar realisiert werden.

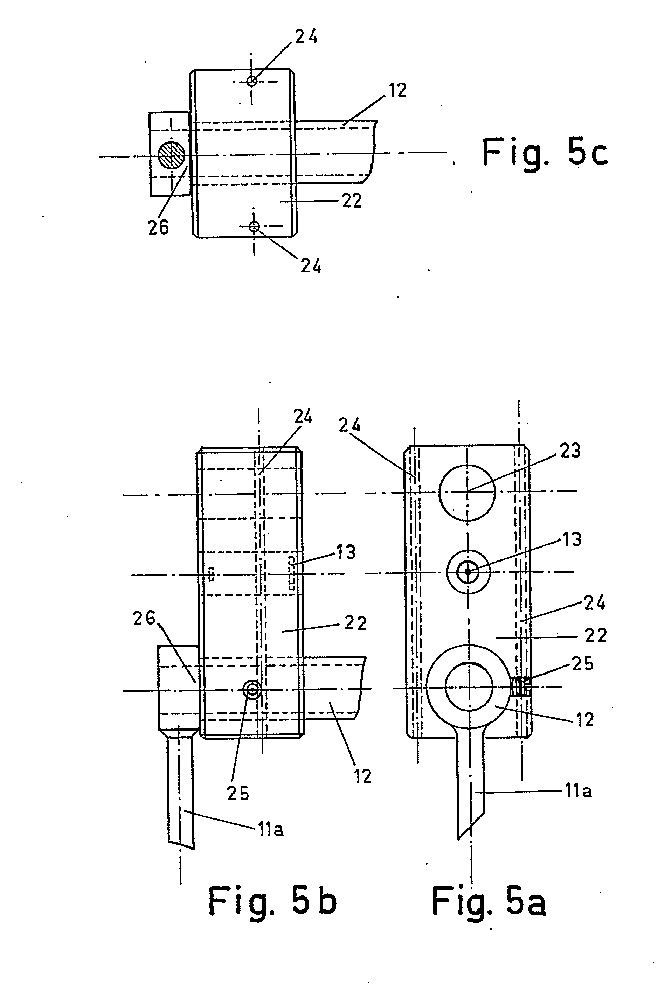

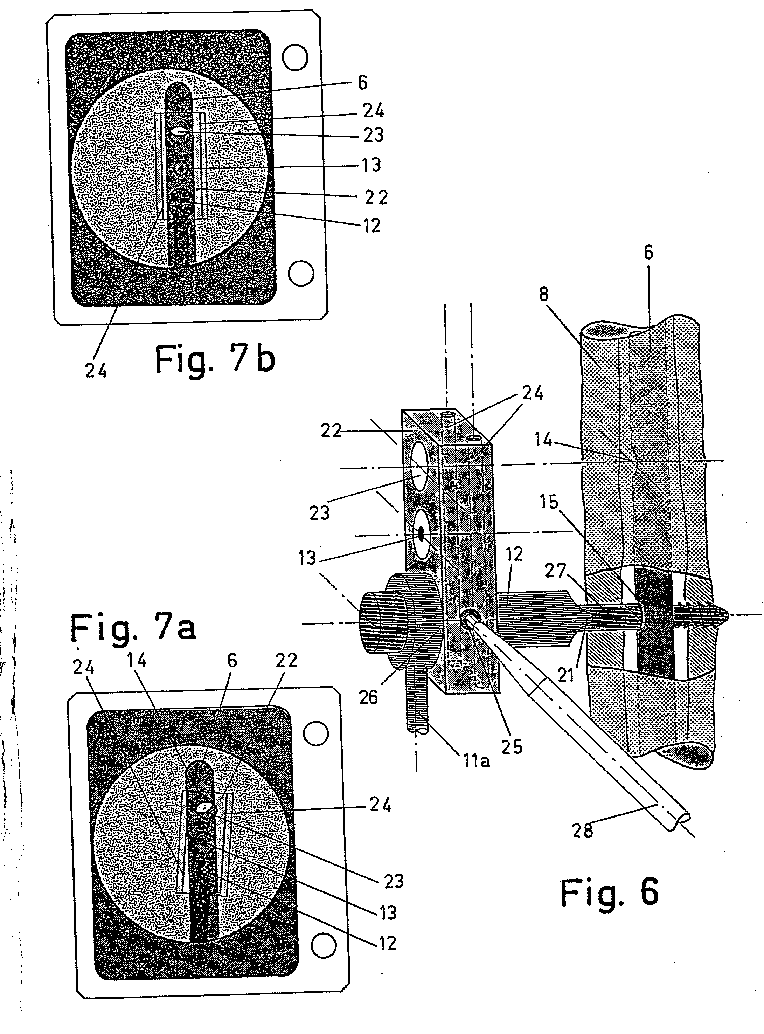

Eine weitere verbesserte Ausführungsform des erfindungsgemässen Visierinstrumentes 10 ist in den Figuren 5 und 6 dargestellt. Die Figuren 5 a bis c zeigen von drei Seiten den Aufbau dieser das Setzen der zweiten Verriegelungsschraube weiter vereinfachenden Ausführungsform. Der mittels der Drehgelenkverbindung 26 schwenkbar um die Bohrhülse 12 angeordnete und mittels der Feststellschraube 25 fixierbare, quaderförmige Block 22 trägt neben dem Visier 13 eine für das Setzen der zweiten Verriegelungsschraube bestimmte Bohrbüchse 23. Der Abstand zwischen den Zentren der Bohrbüchse 23 und der Bohrhülse 12 entspricht dabei genau dem Abstand der Zentren der beiden Querbohrungen 14, 15 des Verriegelungsnagels 6.

Das Visier 13 ist zwischen der Bohrbüchse 23 und der Bohrhülse 12 angeordnet und im übrigen gleich aufgebaut wie in der bereits beschriebenen Ausführungsform der Erfindung.

Um den Block 22 im Strahlenfeld 9 der Röntgenapparatur parallel ausrichten zu können ist dieser aus einem strahlendurchlässigen Material, beispielsweise einem Kunststoff gefertigt und weist zwei zur Zeichenebene der Figur 5a parallel verlaufende, strahlenundurchlässige Metalldrähte 24 auf.

Der technische Ablauf des Setzens der zweiten Verriegelungsschraube mit Hilfe dieser verbesserten Ausführungsform der Erfindung ist aus den Figuren 6 und 7 ersichtlich.
Figur 6 zeigt das Visierinstrument 10 nach erfolgtem Setzen der ersten Verriegelungsschraube gemäss der bereits oben beschriebenen Operationstechnik. Das dazugehörende Monitorbild zeigt typischerweise eine Anordnung wie in Figur 7a dargestellt, d.h. die im Block 22 eingelassenen Metalldrähte 24 sind noch nicht parallel zum Verriegelungsnagel 6 ausgerichtet, sondern schliessen einen Winkel mit diesem ein. Entsprechend liegen die Abbildungen der Bohrbüchse 23 und des zweiten Verriegelungsloches 14 nicht übereinander. Der aus Kunststoff bestehende Block 22 ist zwar im wesentlichen strahlendurchlässig, doch lassen sich in der Praxis seine Konturen ganz schwach auf dem Monitorbild erkennen.
Der Chirurg braucht nun lediglich den Block 22 so lange um die starr mit dem Knochen 8 verbundene Bohrhülse 12 zu drehen, bis er im Monitorbild die Referenz-Metalldrähte 24 parallel zum Verriegelungsnagel 6 ausgerichtet hat und den Block 22 in dieser Position mittels der Feststellschraube 25 zu fixieren.
Sowohl das Ausrichten, als auch das Fixieren des Blockes 22 kann mittels eines gewöhnlichen Sechskantschraubenziehers 28 erfolgen, der in den Sechskanteingriff der Feststellschraube 25 eingeführt wird, wie dies in Figur 6 dargestellt ist.
Bedingt durch die Divergenz des Strahlenfeldes 9 (Fig. 4a) werden sowohl die Bohrbüchse 23, als auch das zweite Verriegelungsloch 14 nicht kreisförmig, sondern elliptisch auf dem Monitor abgebildet. Für das Ausrichten des Blockes 22 spielt dies jedoch keine Rolle, da einzig der Parallelität der Referenzmetalldrähte 24 mit dem Verriegelungsnagel 6 Beachtung zu schenken ist.

Nach Fixierung des Blockes 22 mittels der Feststellschraube 25 kann der Chirurg den Handgriff 11 des Visierinstruments 10 loslassen, so dass er beide Hände frei hat um die zweite Verriegelungsschraube zu setzen. Dazu wird zuerst eine in der Zeichnung nicht dargestellte Gewebeschutzhülse durch die Bohrbüchse 23 gesteckt, in welche dann die 4,5 mm Bohrbüchse eingeführt werden kann.

Der weitere Ablauf entspricht demjenigen des Setzens der ersten Verriegelungsschraube.

Durch diese zeitsparende und präzisionserhöhende Ausführungsform der Erfindung gelingt es die bereits reduzierte Röntgendosis nochmals auf etwa die Hälfte zu senken.

Patentansprüche

1. Visierinstrument (10), insbesondere für chirurgische Zwecke, mit einem Handgriff (11) und einer damit verbundenen Bohrhülse (12), gekennzeichnet durch ein mit der Bohrhülse (12) in definierter Lage verbundenes Visier (13), dessen Position, ebenso wie diejenige der Bohrhülse (12) zwischen einer Strahlenquelle (3) und einem Strahlenempfänger (2) durch einen Wandler (4) sichtbar dargestellt und in kontrollierter Weise verändert werden kann.

2. Visierinstrument (10) nach Anspruch 1, dadurch gekennzeichnet, dass die optische Visierlinie (20) des Visiers (13) und die Achse (18) der Bohrhülse (12) in einer Ebene liegen und sich vorzugsweise im Endlichen schneiden.

3. Visierinstrument (10) nach Anspruch 1 oder 2, dadurch gekennzeichnet, dass der Abstand zwischen Bohrhülse (12) und Visier (13) dem Abstand der Zentren der beiden Querbohrungen (14,15) des Verriegelungsnagels (6) entspricht.

4. Visierinstrument (10) nach einem der Ansprüche 1 bis 3, gekennzeichnet durch einen zusätzlichen, aus strahlendurchlässigem Material bestehenden, in die Bohrhülse (12) einführbaren Stift (16), der an seinem einen Ende einen aus strahlenundurchlässigem Material bestehenden Referenzkörper (17), vorzugsweise von kugelförmiger Gestalt, aufweist.

5. Visierinstrument (10) nach einem der Ansprüche 1 bis 4, dadurch gekennzeichnet, dass das Visier (13) schwenkbar um die Bohrhülse (12) angeordnet ist, vorzugsweise mit Einrastmitteln, die eine Verschwenkung um 90° erlauben.

6. Visierinstrument (10) nach einem der Ansprüche 1 bis 5, dadurch gekennzeichnet, dass das Visier (13) in einem mittels eines Drehgelenkes (26) um die Bohrhülse (12) drehbaren, vorzugsweise mit einer Feststellschraube (25) fixierbaren Blockes (22) angeordnet ist.

7. Visierinstrument (10) nach Anspruch 6, dadurch gekennzeichnet, dass der das Visier (13) tragende schwenkbar um die Bohrhülse (12) angeordnete Block (22) eine Bohrbüchse (23) für das Setzen der zweiten Verriegelungsschraube aufweist, wobei der Abstand zwischen den Zentren der Bohrbüchse (23) und der Bohrhülse (12) dem Abstand der Zentren der beiden Querbohrungen (14,15) des Verriegelungsnagels (6) entspricht.

8. Visierinstrument (10) nach Anspruch 7, dadurch gekennzeichnet, dass das Visier (13) zwischen der Bohrbüchse (23) und der Bohrhülse (12) angeordnet ist.

9. Visierinstrument (10) nach einem der Ansprüche 6 bis 8, dadurch gekennzeichnet, dass der das Visier (13) tragende, strahlendurchlässige Block (22) strahlenundurchlässige Referenzmittel (24), vorzugsweise Metalldrähte, aufweist, welche eine parallele Ausrichtung des Blockes (22) zur Längsachse des Verriegelungsnagels (6) gestattet.

10. Visierinstrument (10) nach einem der Ansprüche 1 bis 9, dadurch gekennzeichnet, dass das Visier (13) zwei in verschiedenen Ebenen liegende Bilder aufweist, welche beim Zielvorgang zur Deckung oder Ergänzung gebracht werden.

11. Verwendung des Visierinstruments (10) nach einem der Ansprüche 1 bis 10, für die Orthopädie, vorzugsweise die Verriegelung der Marknagelung, Arthroskopie, Wirbelsäulenchirurgie und Punktion.

12. Verwendung nach Anspruch 11 für Ausbildungszwecke, dadurch gekennzeichnet, dass die Strahlenquelle (3) sichtbares Licht emittiert und der Strahlenempfänger (2) aus einer Videokamera besteht.

13. Verfahren zur kontrollierten Durchdringung eines Materials (6) in einer gewünschten Richtung mittels eines Werkzeuges (1) unter Ausschluss von chirurgischen Verfahren, dadurch gekennzeichnet, dass das Arbeitsfeld, das Material (6), das Werkzeug (1) und das als Führung für das Werkzeug (1) dienende Visierinstrument (10) zwischen eine Strahlenquelle (3) und einem Strahlenempfänger (2) gebracht wird und das Visier (13) des Zielinstruments (10) mittels optischer Kontrolle über einen an den Strahlenempfänger (2) angeschlossenen Wandler (4) mit Monitor (5) während der Durchdringung des Materials (6) parallel zum Strahlenfeld (9) ausgerichtet wird.

# Fig. 1

Fig. 2a

Fig. 2b

Fig. 3b

Fig. 3a

Fig. 4a

Fig. 4b

Fig. 5c

Fig. 5b

Fig. 5a

Fig. 7b

Fig. 7a

Fig. 6